# EUROPEAN PATENT APPLICATION

(11) **EP 2 295 099 A1**
(43) Date of publication of application: **16.03.2011**
(21) Application number: 09170149.0
(22) Date of filing: 14.09.2009
(51) Int. Cl.: A61M 5/32

(54) **Unit for safe unsheathing and re-sheathing of syringe needles**

(71) Applicant: BMO-Medical Limited, 4886 Grimstad (NO)
(72) Inventor: Andreassen, Arne, Kjell, 4870, Fevik (NO); Brannfjell, Tor, Finn, 4877, Grimstad (NO)
(74) Representative: Acapo AS

(57) **Abstract**

The present invention relates to a unit (10) for safe handling of needles of syringes, including unsheathing and/or re-sheathing a sheath (30) covering the syringe needle, the unit (10) comprises retaining means (15) for locking, temporarily storing and releasing the stored sheath (30), and means for manipulating said retaining means (15) to provide and/or suspend the locking effect on the sheath (30).

Said means (15) for locking, temporarily storing and releasing the stored sheath (30) comprises a receiver body (17), movable in a first direction with respect to a housing (18,21) against a force of a spring (24), and at least one locking surface (38), movable in a second direction with respect to the direction of movement of the receiving body (17), moving the locking surfaces (38) into or out of engagement with the inserted sheath (30), such latter movement being caused by the movement of the receiving body (17).

## Description

### Technical Field of the Invention

The present invention relates to a unit for safe handling of syringe needles, including unsheathing and/or re-sheathing a sheath covering the syringe needle, the unit comprises retaining means for locking, temporarily storing and releasing the stored sheath, and means for manipulating said retaining means to provide and/or suspend the locking effect on the sheath.

### Background for the Invention

A syringe, for example for medical use, is used for injection a solution or a liquid into or withdrawing blood or body fluids from a body. From a safety point of view it is important that the user, and/or the patient on which medication or liquid withdrawal is administered, is not accidentally pricked by the needle of the syringe. It is also imperative that the needle remains sterile prior to insertion into the human body.

The conventional syringes has a tubular sheath placed on the syringe needle, securing that the needle remains sterile and preventing accidental pricking. Such sheath must be removed prior to insertion, requiring normally two hands for unsheathing or re-sheathing.

Hence, there is a need for equipment enabling removal of a sheath with one hand. It has previously been proposed to use an apparatus permitting a person to unsheathe and re-sheath the needle with the use of only one hand.

US 5,242,426 describes an apparatus for permitting a person to unsheath and re-sheath the needles of hypodermic syringes, catheters, etc., with the use of only one hand. The apparatus permits free use of the individual's other hand when such is required, e.g. when restraint of the patient is necessary in order to properly and safely administer an injection from a hypodermic needle or the like. The apparatus securely holds and positions a medical needle sheath during such times that a medical needle is removed from and re-inserted into the sheath.

US 5,067,949 describes a means for protecting the user of a medical syringe from being pricked by its needle when unsheathing or re-sheathing the needle. The means has an upper member mounted atop a disposable container. A vertical passage through the upper member empties through a hole into the container. The passage has a coned entry mouth enabling the user to guide the sheathed needle end of a syringe into the passage. A cam in the upper member is pivotably arranged to hold the sheathed needle in the passage, so that the user may pull the on the syringe and separate its needle end from the sheath, or later, the user may re-insert the used needle end of the syringe into the cammed sheath and, upon twisting the syringe, separate it from the needle to allow the separated re-sheathed needle to drop into the container following release of the cammed condition.

US 5,308,582 describes a hypodermic syringe uncapping device which includes a housing enclosing two opposed, pivotal arms adapted to grip a cap protecting a hypodermic syringe needle. Upon insertion through a hole in the housing, the capped needle is forced against a movable plunger whose lower end engages one of a number of pins extending from the surface of a rotatable cam located between the arms. Rotation of the cam thus induced allows the arms to pivot towards each other under the urging of an arm-connecting spring, causing the arms to grasp the cap and thereby allow subsequent withdrawal of the needle. When the needle is re-inserted in the cap and the cap is again pushed against the plunger, the cam is rotated still further, forcing the arms apart and permitting the cap to be withdrawn from the device.

There is a need for an improved apparatus which is safe to use, eliminating the risk for being pricked by the needle, such apparatus also being reliable and simple in construction, easy to operate, and providing improved locking effect on the sheath. Further there is a need for an apparatus which does not occupy a large space or which may be handheld.

### Summary of the Invention

An object of the present invention is to provide a solution which is safe and simple to handle, reducing the risk of being pricked when sheathing or unsheathing a needle on a syringe.

A further object of the invention is to provide a unit enabling one-hand unsheathing or re-sheathing, for example a sheath of plastic materials of a syringe needle, without having to touch the sheath and/or the unit.

Another object of the present invention is to provide a unit which is easy to use and being reliable in use.

A still further object of the present invention is to provide a unit making the possible only to use one hand when sheathing and/or unsheathing a needle on a syringe.

Yet another object is to provide a unit which is purely mechanical, not requiring other energy sources than the force provided by the user, inserting or removing the sheathed or un-sheathed syringe needle into or out of the unit.

A still further object of the present invention is to provide a unit which is maintenance free.

A still further object is to provide a unit not requiring special cleaning as a consequence of normal use, since no direct contact bye the user is necessary.

According to the present invention, said objects are achieved by a unit as further defined by the independent claim. Different embodiments of the unit according to the invention are defined in the dependent claims.

According to one embodiment of the invention, the means for locking, temporarily storing and releasing the stored sheath comprises a receiver body, movable in a first direction with respect to a housing against a force of a spring, and at least one locking surface, movable in a second direction with respect to the direction of movement of the receiving body, moving the locking surfaces into or out of engagement with the inserted sheath, such latter movement being caused by the movement of the receiving body.

According to one embodiment, said movement of the receiver body may be linear, while the movement of said at least one locking surface preferably may be perpendicularly towards the sheath.

Said at least one locking edge or surface may extend through an opening or slot in the wall of the receiver body, providing a lateral retaining forced on the sheath, thereby locking sheath against axial movement.

At least two locking surfaces, diametrically arranged with respect to the receiver body, may be used.

Further, the locking surfaces of the locking means may be configured in such way that at least four contact points against the sheath are formed. Preferably, the locking means may be in the form of knife edged surface.

Each locking surface may be attached to an arm, said arm being pivotably arranged in such way that the free end of each arm is forced laterally towards and into the tubular body by means of a skewed contact walls arranged in the housing, causing a wedging effect so that the locking effects of the knives are further enhanced when the syringed is pulled outwards.

According to another embodiment, a surface on the receiver body may be provided with groove or recess while a corresponding surface of a knob of the housing is provided with a pin arranged on a knob movably arranged in said groove, the groove being configured in such way the locking effect of the receiver body may be suspended by pushing the receiver body inwards against the action of the spring force and then release the inwards pressure.

The unit may preferably, but not necessary, be fixed inside a housing configured to be attached to a supporting surface, the unit forming an integral part of the housing.

### Short Description of the Drawings

One embodiment of the present invention will be described below referring to the accompanying drawings, wherein:
Figure 1 shows schematically in perspective a partly exploded view of a unit for secure handling of syringes;
Figure 2a shows the cassette in exploded view in perspective, seen from above, while Figure 2b shows the cassette in exploded view in perspective, seen from below;
Figure 3 shows schematically in perspective a partly exploded view corresponding to the view shown in Figure 1, where a shielding cover is removed;
Figure 4 shows schematically in perspective the lower part of the unit shown in Figure 1, where a syringe is inserted into to receptacle;
Figure 5 and 6 show schematically in perspective two extreme positions of the locking and retaining element with the cover removed, where Figure 5 shows a position where the ends of the pivotable arms in a locking apposition, while Figure 6 show the opposite position where the holding effects of the arms are suspended;
Figure 7 shows the bottom element of the cassette according to the present invention;
Figure 8 shows the top element of the cassette with the locking element and the arms, seen from below;
Figure 9 shows a view of the receiver body 17, seen from above; and
Figures 10-21 show various stages in the manipulating process of the receiver body for securing locking and/or release of the sheath in the receiver body.

### Detailed Description of the Invention

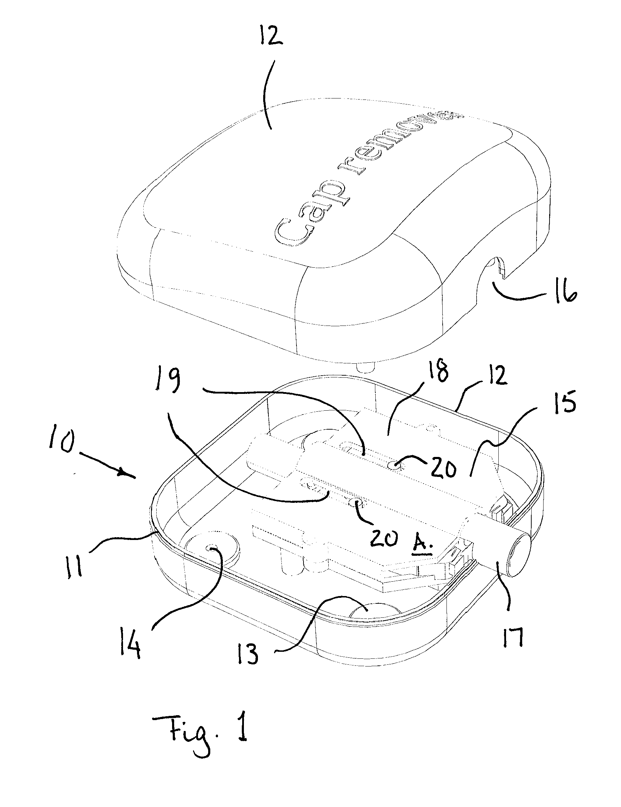

Figure 1 shows schematically in perspective a partly exploded view of a unit 10 for secure handling of syringes (not shown). The unit 10 comprises a bottom tray 11 and a top cover 12, configured to be assembled to a single body, e.g. by means of screws, snapping elements or the like. The bottom tray 11 may be provided with reinforced areas 13 provided with holes 14 for enabling fixing of the unit 10 to a supporting surface (not shown) by means e.g. of screws (not shown) or the like.

When assembled, the bottom tray 11 and the top cover 12 form a hollow body containing a closed locking and retaining cassette 15 which will be described in further detail below. Further one side wall in the bottom part 11 and the corresponding side wall of the top part 12 are provided with recesses, forming an opening 16 when assembled, the opening 16 being configured for receiving one end of an axially displaceable, outwardly protruding tubular receiver body 17 of the cassette 15. As will be described in further detail below, the cassette 15 is fixed to the bottom part 11, e.g. by means of screws, glue, snapping means or the like. The cassette 15 comprises in principle a base 21, a movable sledge or body 29, pivotably arranged arms 27, a spring 24 and a cover 18. The cassette 15 as such and the functioning of the cassette 15 will be described in further details below.

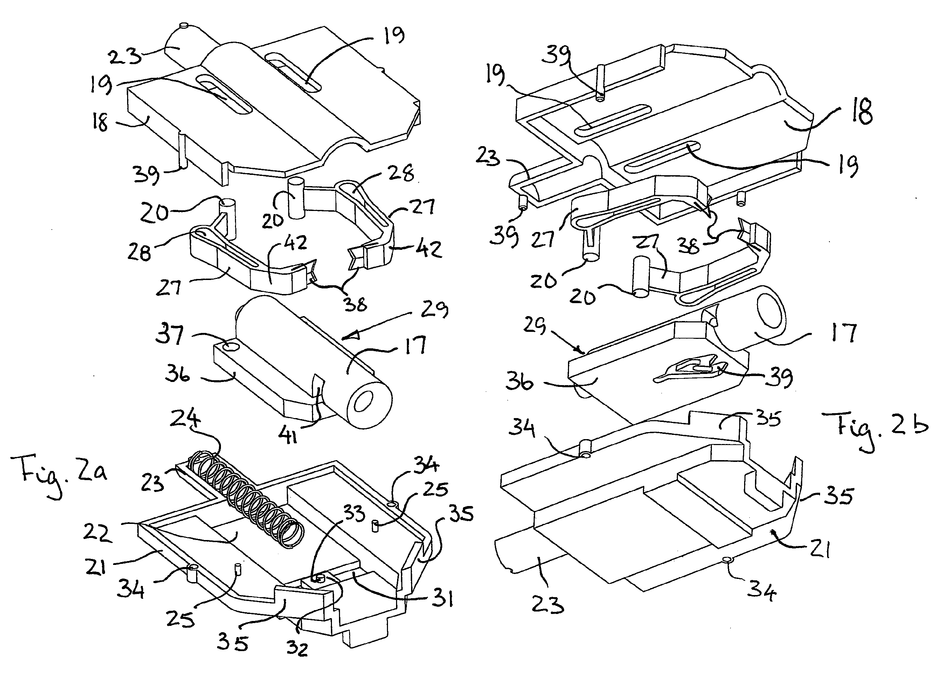

Figure 2a shows the cassette 15 in exploded view in perspective, seen from above, while Figure 2b shows the cassette 15 in exploded view in perspective, seen from below. As indicated the in Figures 2a and 2b, the cassette 15 comprises a base 21, a spring 24, a receiver body 17 for receiving and temporarily storing a sheath 30, arms 27, and a lid 18.

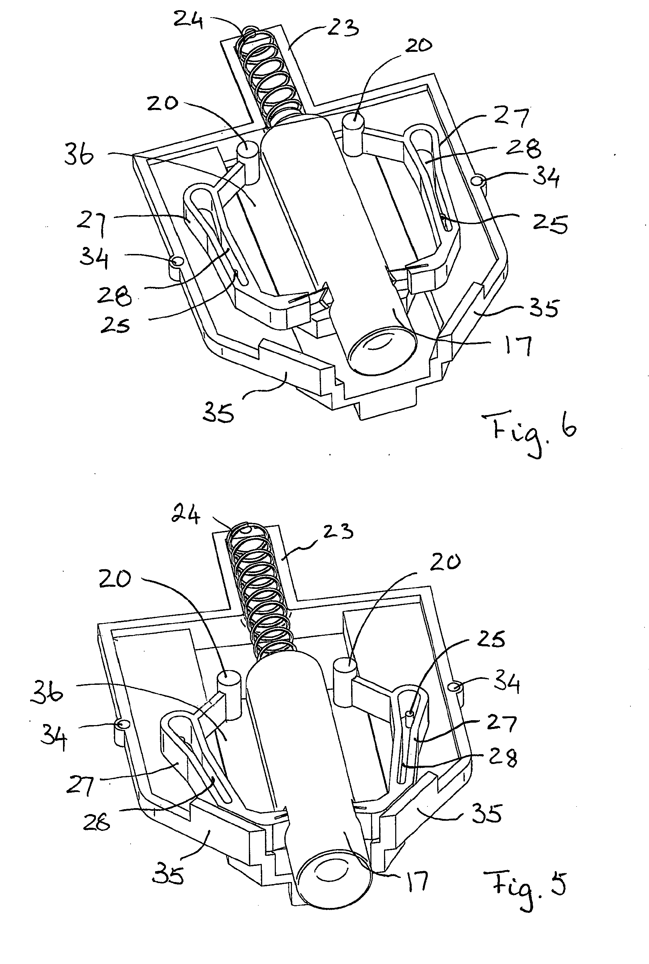

The base 21 is provided with a recess 22, axially arranged with respect to the opening 16 and a semi-cylindrical, cradle shaped part 23. The cradle shaped part 23 is aligned with a corresponding semi-cylindrical cradle shaped part 23 on the lid 18, thus together forming a house for the spring 24. Further, the base 21 is provided with at least two upwards extending guide pins 25. The function of said guide pins 25 will be described in further detail below in conjunction with Figures 5 and 6. Further, in the axially extending recess 22, a transverse recess 31 is arranged. A knob 32, provided with a pin 33, extending upwards from the knob 32. The knob 32 is movable in transverse direction with respect to the recess 22. Further, the base 21 is provided with holes 34 for receiving corresponding pins or screws 39 on the lid 18.

At the front end of the recess 22, i.e. the end adjacent the opening 16 of the unit 10, the base 21 is provided with upwards projecting, skewed walls 35, configured in such way that the skewed walls 35 are pointing towards each other in direction towards the opening 16.

The movable receiver body 17 is in the form of a cylindrical, hollow part with an opening, configured to receive and store the sheath 30. The hole in the cylindrical part extends in axial direction of the body 17. The receiver body 17 is further provided with a lower sledge portion 36, configured to be slidably arranged for movement back and from in the recess 22, subjected to the effect of the spring force of the spring 24. The front end of the sledge portion 36 is given a skewed shape, corresponding to the skewed walls 35. At the opposite end of the sledge 36, the sledge 36 is provided with holes 37. The holes 37 are configured to receive pivot pins 20, arranged at the end of the arms 27.

As specified above, the arms 27 are at one end provided with a pivot pin 20. Said pivot pin 20 extends out from the arm 27 both upwards and downwards. Further, the opposite end of each arm 27 is provided with knife edge(s) 38, configured to create the required locking effect for locking a sheath 30 inserted into the receiver body 17. Further, each arm 27 is provided with a slot 28, configured to receive the corresponding pin 25 on the base 21. In general each arm is given a more or less U-shape, having skewed intermediate portions between the slotted part and the pivot pin 20 on the one end and between the slotted part and the knife(s) 38 on the other end. In order to ease relative movement of the arms 27 with respect to the pin 25, the end of the slot 28 adjacent the pivot pin 20 is wider than the remaining part of the slot 28.

The top cover 18 is provided with two slots 19, configured to receive the upper end of the pivot pin 20, allowing the pivot pins 20 to slide back and from in the slots 19 when the receiver body 17 is moved axially back and from. Further, the top cover 18 is given a portion having a cylindrical dome shaped section, adapted to the shape of the upper end of the receiver body 17 and skewed end surfaces. Further, the top cover 18 is provided with downwards projecting pins 39 configured to be introduced into corresponding holes in the base 21 for locking the two parts 18,21 together.

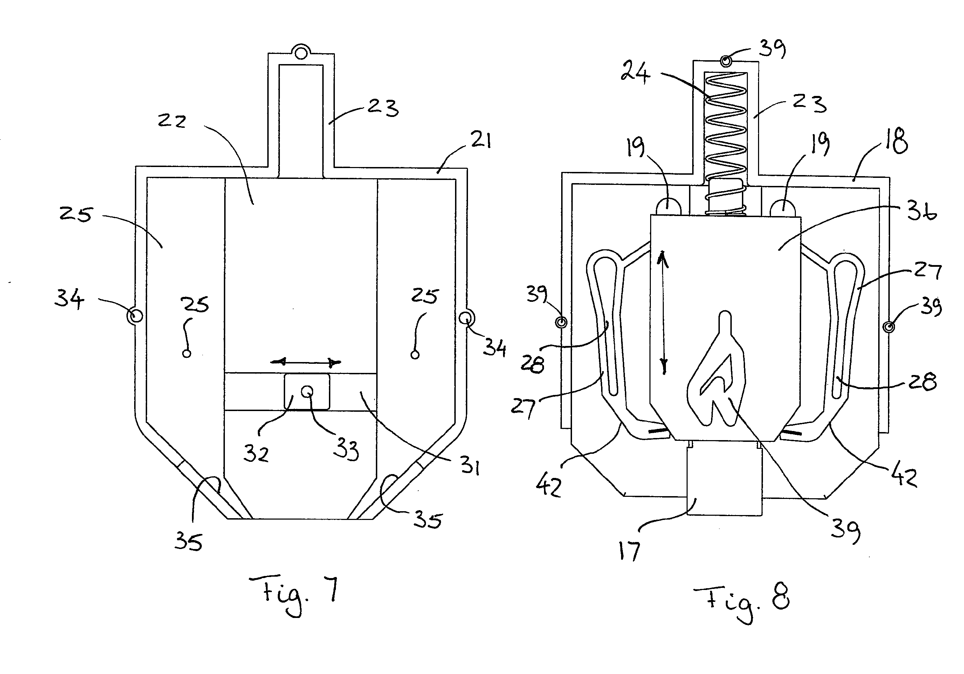

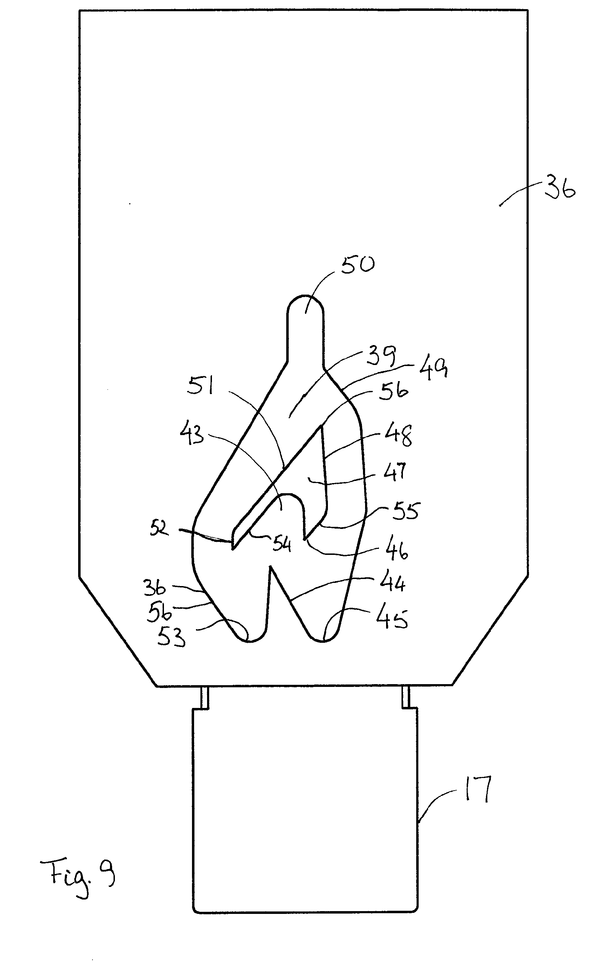

As shown in Figure 2b, the sledge 36 is provided with a recess 39 in its bottom surface, configured to cooperate with the upwards projecting pin 33 on the knob 32, securing that the sledge 36 may be locked in either of two positions. These locking functions will be described below in conjunction with Figures 8 and 9.

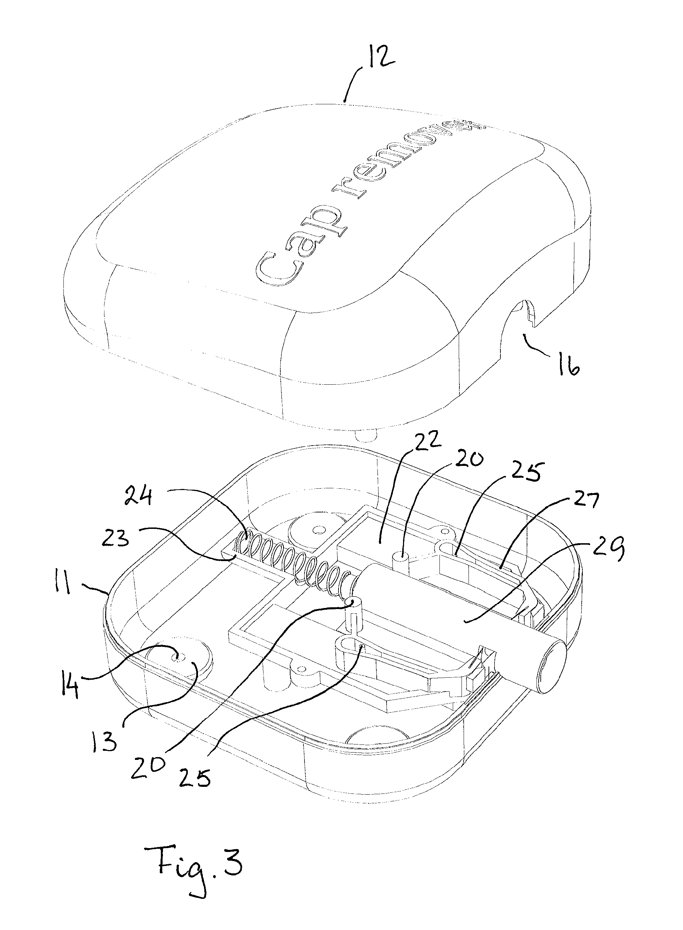

Figure 3 shows schematically in perspective a partly exploded view of the unit 10 corresponding to the view shown in Figure 1, where the shielding cover or lid 18 is removed. As shown in Figure 3, the cassette 15 is provided with a base 21, which may form an integral and monolithic part of the bottom tray 11. Alternatively, the base 21 may be fixed to the bottom tray 11 by means of gluing, screws, snapping, or the like.

According to the embodiment shown in Figure 3, the two arms 27 are pivotably arranged on the sledge 36, rotating around the pivot pins 20 at the end of each arm 17. Each pin or shaft 20 is pivotably arranged in the corresponding recess or hole 37 at one end of the sledge 36. Further, as indicated above, each arm comprises at least one knife edge 38, configured to be forced into contact with the sheath when inserted into the receiver body 17 through slots 41 in said body 17, reaching the interior axial bore of the receiver body 17. The arms 27 further comprises elongate slots or recesses 28, into which the guide pins 25 are arranged, so that when the receiver body 17 and the sledge 36 is moved in axial direction, the arms 27 move with respect to the pins 25, thereby causing a sideways movement of the arms and consequently the knife edges, which projects into the slot 41, into or out of locking contact with respect to the inserted sheath 30.

At the end of the base 21 facing the opening 16, the base is provided with two skewed surfaces 35, projecting upwards from the base, sideways displaced with respect to the opening 16. Said skewed surfaces 35 is configured to cooperate with corresponding skewed surfaces 42 on the arms 27, contributing to and enhancing the locking effect of the knife edges 38 on to the inserted sheath 30. Since the arm 27 is pivotably hinged at one end, and due to the slots or recesses 28 in the sideways projecting middle part of arms 27, an axial movement of the receiver body 17 with the sledge body 36 will cause a motion of the opposite end of the arm 27 out of or into the slot 41, producing or suspending the locking effect on the sheath 30. The grooves 28 are configured to cooperate with the pins 25 so as to function as a guide for the axial displacement of the moveable part 29 of the locking and retaining cassette 15. The design, configuration and the function of the various elements of the retaining and locking cassette 15 will be described in further detail below.

As further indicated in Figure 3, the receiver body 17 is configured to move back and from in the recess 22, the arms 27 moving together with the receiver body 17. Such movement is guided by the pivot pins 20, sliding back and from in the slots 19 in the top cover 18 of the unit 10. The axial movement of the receiver body 17 also causes the arms 27 to pivot around the pivot 20, such pivoting movement being caused by the stationary guide pins 25 sliding in the slots 28 in the arms 27, moving the edges 38 out of or into the slots 41 in the receiver body. In such way, a locking or releasing effect of the sheath 30 is provided.

One end of the tubular body 17 is configured for receiving a sheath 30 and locking and retaining such sheath 30 when the syringe is administered. For such purpose the tubular body 17 is provided with an axially arranged, tubular hole and a tapered mouth, the dimensions of such recess being chosen so that the unit 10 may be used for most of the commercially available syringes with sheaths 30, independent of supplier.

At the opposite end, the tubular body is provided with an axially arranged tubular recess, intended to receive one end of the spring 24, securing proper attachment of such spring 24 to the receiver body 17.

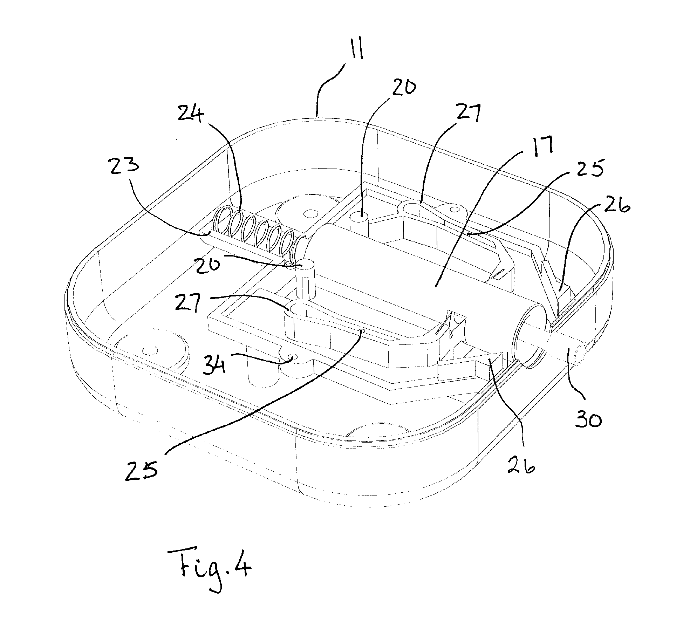

Figure 4 shows schematically in perspective the lower part of the unit shown in Figure 1, where a sheath 30 is inserted into the receiver body 17 and where sheath 30 is in locked position, the receiver body 17 being in its outer extreme position, locking the sheath and storing it temporarily, until the needle of the syringe is reintroduced into the sheath 30 and the locking effect of the locking mechanism again being released by a slight movement of the receiver body 17, thus enabling the operator to remove a sheathed syringe out of the unit 10.

Figure 5 and 6 show schematically in perspective two extreme positions of the locking and retaining receiver body 15 with the cover 18 removed. Figure 5 shows a position where the free ends of the pivotable arms 27 are in a locking position, while Figure 6 show the opposite position where the holding and locking effects of the arms 27 are suspended. As seen in the Figure 5 the skewed surfaces 35 on the base 21 and the skewed surfaces 42 on the arms contribute to the forcing of the knife edges 38 of the arms 27 into the slot 41 in the receiver body 17.

Figure 7 shows the bottom element 21 of the cassette 15, seen from above. As shown, the bottom element 21 has a recess 22, configured to receive the movable sledge 29 of the receiver body 17. The recess 22 extends in one axial direction, coinciding with the cradle shaped body 23 at the rear end of the bottom element 21. As shown a knob 32 provided with an upwards projecting pin 33 is arranged in a second recess 31 which extends in traverse direction with respect to the recess 22, the knob 32 being configured to move back and from in the recess 31. Said pin 33 is configured to be in engagement with a recess 39 in the lower surface of the movable sledge 36 on the receiver body 17.

Figure 8 shows the receiver body 17 and the cover 18 seen from below, showing the recesses 19 for guiding the pivots 20, the shape of the recess 39 on the bottom surface of the sledge 36, parts of the arms 27. As shown, the receiver body 17 is in a position where the knife edges 38 on the arms 27 are in the first position, not inserted into the slots 41 and into a central bore in the receiver body 17. The pins or shafts 25 are in the narrow part of the slot 28. In this position substantially no or little spring force acts on the sledge 36.

Figure 9 shows a view of receiver body 17 seen from below, indicating the various surfaces and seats referred to below. In general, the lower surface of the sledge 36 of the receiver body 17 is provided with a recess 39 being provided a centrally arranged middle part 47 projecting outwards from the recess 39, creating guiding surfaces and recesses. The surface of the sledge 36 of the receiver body 17 and the outer surface of the centrally arranged middle part 47 co-inside.

In the following the function of the unit 10 will be described in further details, referring to Figures 9-21.

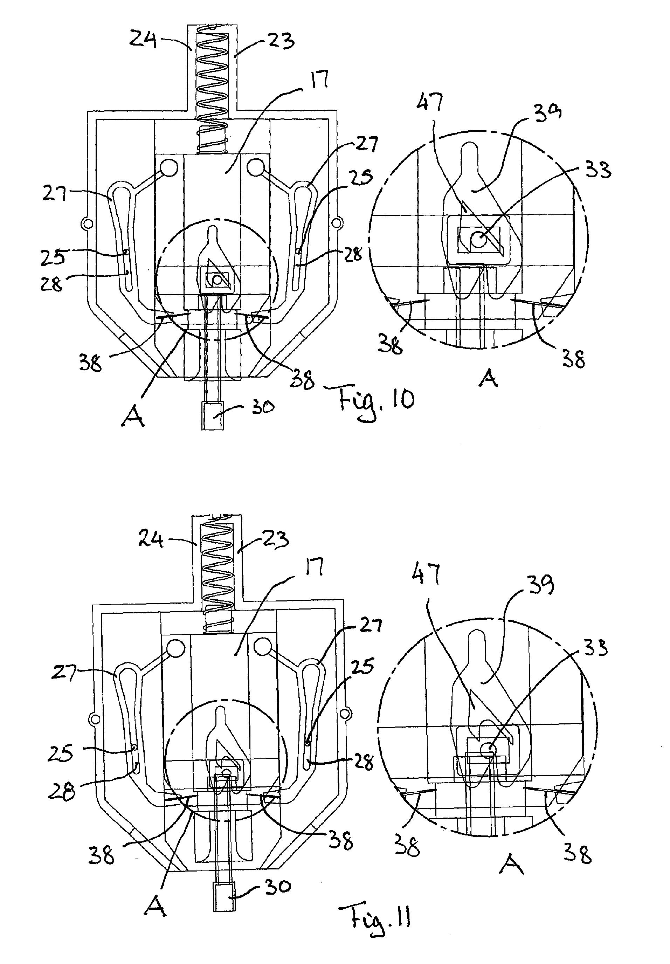

Each of the Figures 10-21 show a view seen from above of the cassette 15 with the cover 18 removed. Each Figure shows the positions of the arms 27 and the locking knife edges 38 during the various stages. Further, each Figure shows an enlarged view of the part of the unit marked with a circle, showing the various positions of the pin 33 on the knob 32 with respect to the various seats and surfaces of the recess 39.

Figure 10 shows a position where the receiver body 17 is in an open position, ready for receiving the sheath 30. As indicated in Figure 10, the locking knife edges 38 are in its extreme outer position, allowing introduction of the sheath 30 into the central hole in the receiver body 17. In this position, the pin 33 on the movable knob 32 is resting in its extreme outer locking seat 43 in the recess 39. Further, in this position, the guide pins 25 are in a middle position in the slots 28 in the arms 27. The spring 24 is in a compressed position, exerting a spring force on the receiver body 17, thus locking the position of the receiver body 17.

According to Figure 11 the user presses a sheath 30 into the receiver body 17, moving the receiver body 17 inwards so that the pin 33 leaves its seat 43 while the receiver body 17 and the arms 27 are pressed even further into the cassette 15, thereby forcing the knife edges 38 further out of the slot 41, securing free access for the sheath 30 into the receiver body 17. As indicated in the enlarged part of the Figure, the pin 33 hits a skewed surface 44, thus moving the knob 32 sideways towards left on Figure 11.

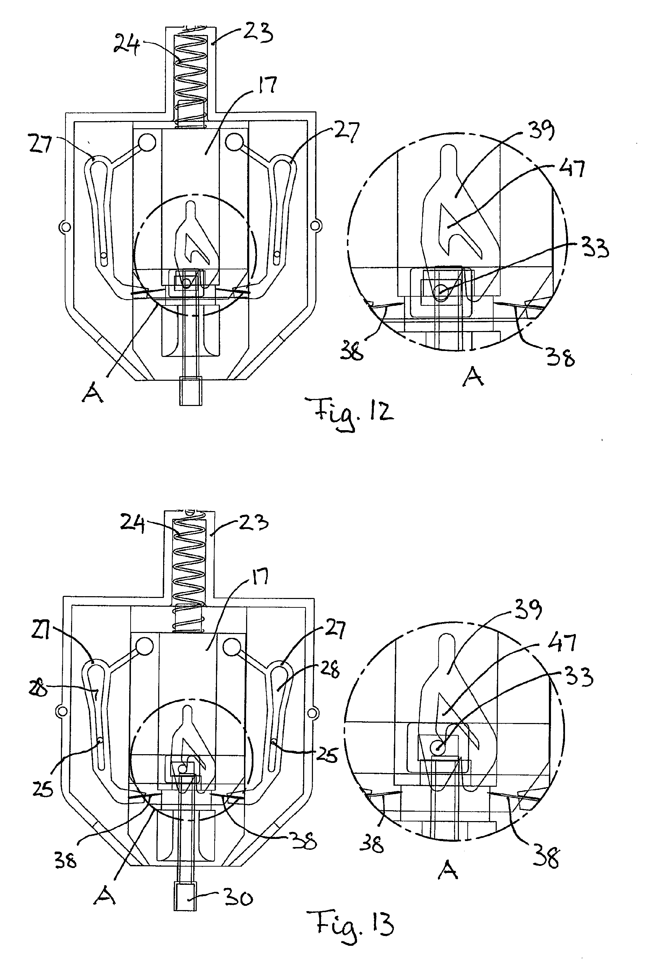

Figure 12 shows a view where the pin 33 on the movable knob 32 is in its extreme, intermediate position, resting against the extreme intermediate seat 45, in which the pin 33 is free off the edge 46 on the central part 47 of the recess 39. As further indicated in the Figure 12, the guide pins 25 are in their extreme end position in the slots 28 in the arms 27.

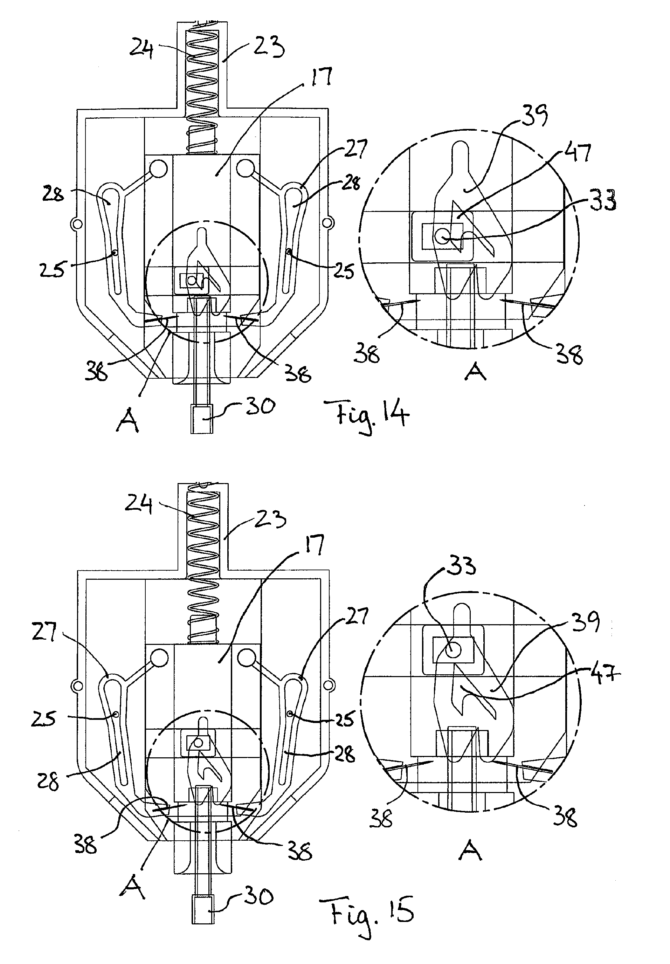

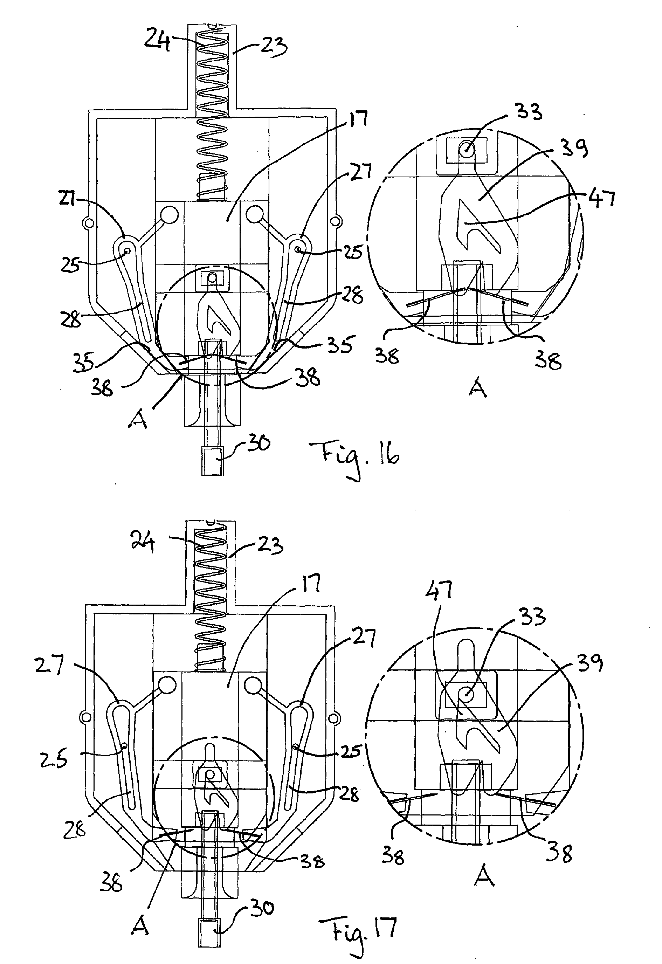

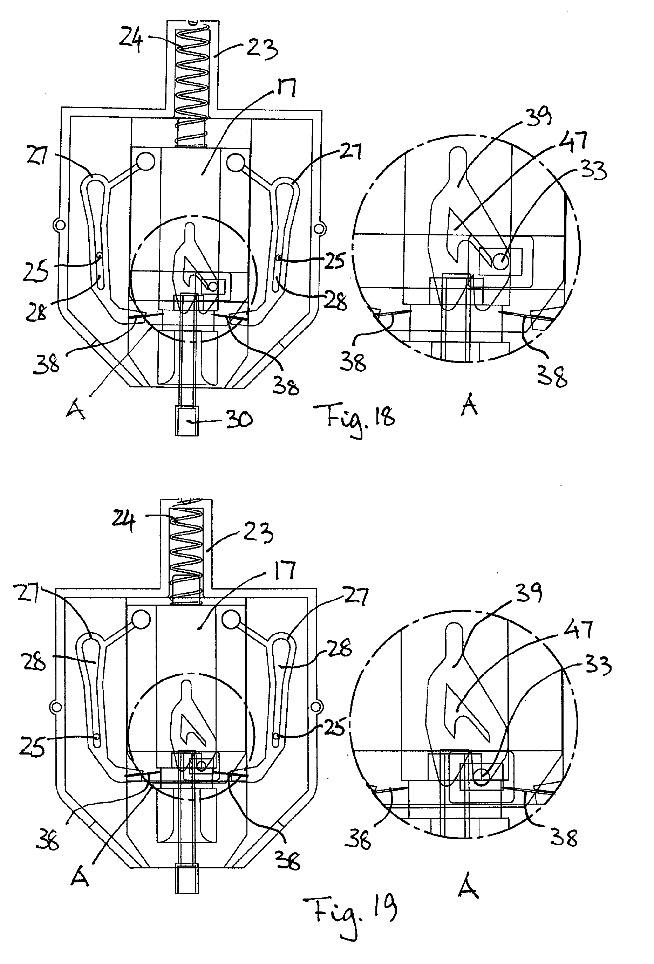

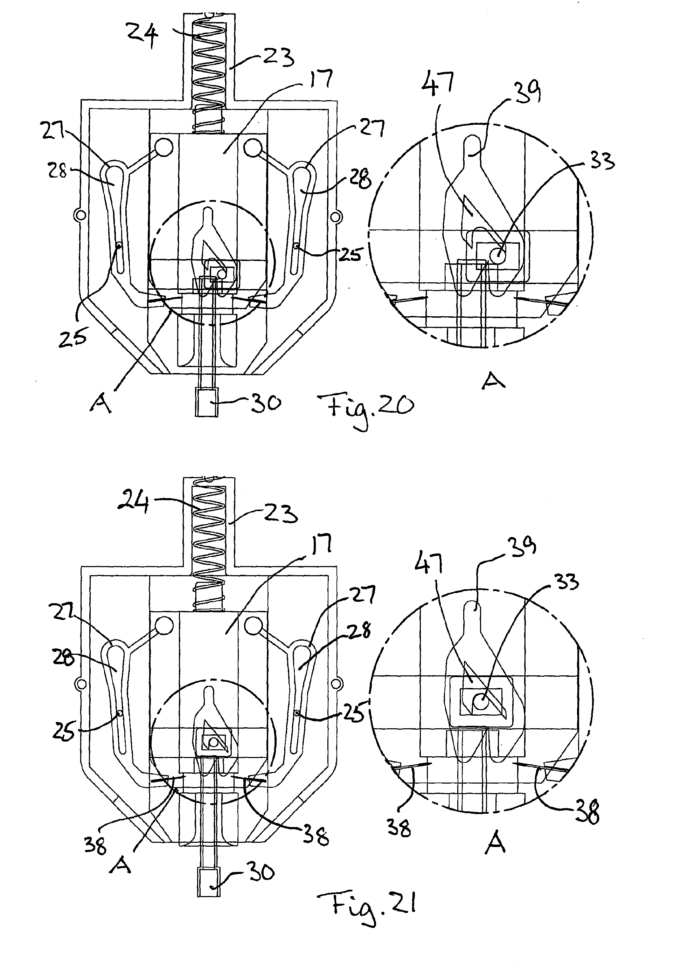

Upon release of the inwards pressure the spring will, as shown in Figure 13, force the receiver body 17 outwards, securing that the pin 33 on the knob 32 will hit the outer skewed surface 55 on the outwards projecting middle part 47, passing the centrally arranged outwards projecting middle part 47 in the recess 39, ref. Figure 13. When the pressure on the receiver body 17 is further released, the pin 33 will as indicated in Figure 14, slide along the surface 48. As shown in Figure 15, the pin will then hit the skewed surface 49 and enter into the opposite extreme seat 50, ref. Figure 16. During the movement between the position shown in Figure 15 and the position shown in Figure 16, the receiver body 17 has moved towards the front end, the skewed surfaces 42 on the arms 27 contacting the skewed walls 35, so that the arms 27 are forced into the slots 41 in the receiver body 17, forcing the knife edges 38 into locking contact with the sheath 30. The receiver body 17 will remain in this extreme end position, exposed by the counteracting spring force, until the user again manipulates the sheath 30 by inserting the needle into the locked sheath, pressing the receiver body 17 inwards against the action of spring force of the spring 24. Such movement will, due to the guide pins 25 in the slots 28 in the arms 27, and due to the pressure induced on the receiver body 17 by the user, retract the knife edges 38 from its locking position in the slot 41. The pin 33 on the knob 32 will then hit the skewed surface 51 on the central, outwards projecting body 47 in the recess 39, ref. Figure 17, allowing the pin 33 to slide along this surface 51, until the pin passes the tip surface 52, ref. Figure 18, whereupon the pin 33 will move further down into a second, temporary seat 53, ref. Figure 19. During such movement, the pin 33 and the knob 32 have been moved slightly inwards (to the left on the Figure when it hits the skewed wall 56. Thereupon, the pin 33 will move in opposite direction from the second temporary seat 53, hitting the skewed surface 54, ref. Figure 20 and sliding back into the first locking seat 43, ref. Figure 21. For the sake of good order, Figure 21 corresponds to figure 10. At this stage the sheath 30 is free to be removed, attached on the needle, while the cassette 15 is ready for receiving a new sheath 30.

According to the present invention a solution being safe and simple to handle is provided, reducing the risk of being pricked when sheathing or unsheathing a needle on a syringe.

Further a unit enabling one-hand unsheathing or re-sheathing, for example a sheath of plastic materials of a syringe needle is provided, enabling operation without having to touch the sheath and/or the unit.

It should also be appreciated that the unit is easy to use and is reliable in use. Further, it will be possible to handle the unit by only using use one hand when sheathing and/or unsheathing a needle on a syringe.

Also, the unit is purely mechanical, not requiring other energy sources than the force provided by the user, inserting or removing the sheathed or un-sheathed syringe needle into or out of the unit.

Further, the unit which is maintenance free and does not require special cleaning as a consequence of normal use, since no direct contact bye the user is necessary.

## Claims

1. Unit (10) for safe handling of needles of syringes, including unsheathing and/or re-sheathing a sheath (30) covering the syringe needle, the unit (10) comprises retaining means (15) for locking, temporarily storing and releasing the stored sheath (30), and means for manipulating said retaining means (15) to provide and/or suspend the locking effect on the sheath (30),
**characterized in that** said means (15) for locking, temporarily storing and releasing the stored sheath (30) comprises a receiver body (17), movable in a first direction with respect to a housing (18,21) against a force of a spring (24), and at least one locking surface (38), movable in a second direction with respect to the direction of movement of the receiving body (17), moving the locking surfaces (38) into or out of engagement with the inserted sheath (30), such latter movement being caused by the movement of the receiving body (17).

2. Unit (10) according to claim 1, wherein said movement of the receiver body (17) is linear.

3. Unit (10) according to claim 1 or 2, wherein the movement of said at least one locking surface (38) is perpendicularly towards the sheath (30).

4. Unit (10) according to one of the claims 1-3, wherein said at least one locking edge or surface (38) extends through an opening (41) in the wall of the receiver body (17), providing a lateral retaining forced on the sheath (30), thereby locking sheath (30) against axial movement.

5. Unit (10) according to one of the claims 1-4, wherein at least two locking surfaces (38) are diametrically arranged with respect to the receiver body (17).

6. Unit (10) according to one of the claims 1-5, wherein the locking surfaces (38) of the locking means are configured in such way that at least four contact points against the sheath (30) are formed.

7. Unit (10) according to one of the claims 1-6, wherein the locking means are in the form of knife edged surface (38).

8. Unit (10) according to one of the claims 1-7, wherein each locking surface (38) is attached to an arm (27), said arm (27) being pivotably arranged in such way that the free end of each arm (27) is forced laterally towards the tubular body (17) by means of a skewed contact walls (35) arranged in the housing (18,21), causing a wedging effect so that the locking effects of the knives (38) are further enhanced when the syringed is pulled outwards.

9. Unit (10) to one of the claims 1-8, wherein a surface on the receiver body (17) is provided with groove or recess (39) while a corresponding surface of a knob (32) of the housing (18,21) being provided with a pin (33) arranged on a knob (32) movably arranged in said groove (39), the groove (39) being configured in such way the locking effect of the receiver body (17) may be suspended by pushing the receiver body (17) inwards against the action of the spring force and then release the inwards pressure.

10. Unit (10) according to one of the claims 1-9, wherein the unit (10) is fixed inside a housing (11,12) configured to be attached to a supporting surface, the unit (10) forming an integral part of the housing (11,12).

## Amended claims

### Amended claims in accordance with Rule 137(2) EPC.

**1.** Unit (10) for safe handling of syringes during unsheathing and/or re-sheathing a sheath (30) covering the syringe needle, the unit (10) comprises housing (18,21) containing a receiving body (17) configured to receive, locking, temporarily storing and releasing the stored sheath (30), and means for manipulating said receiving body (17) to produce and/or suspend the locking effect on the sheath (30) by moving the receiving body (17) in a first direction with respect to the housing (18,21) against a force of a spring (24), and at least one locking surface (38), movable in a second direction with respect to the direction of movement of the receiving body (17), moving the locking surfaces (38) into or out of engagement with the inserted sheath (30) in the receiving body (17), such latter movement being caused by the movement of the receiving body (17)
**characterized in that**. the locking surfaces (38) are configured in such way that the locking surfaces (38) are maintained in locking engagement with the inserted sheath (30) in the receiving body (17) until the locking effect is suspended by a new axial movement of the receiving body (15), and that the locking engagement being intended to be manipulated by the syringe.

**2.** Unit (10) according to claim 1, wherein said at least one locking edge or surface (38) extends through an opening (41) in the wall of the receiving body (17), providing a lateral retaining forced on the sheath (30), thereby locking sheath (30) against axial movement.

**3.** Unit (10) according to claim 1 or 2" wherein at least two locking edges or surfaces (38) are diametrically arranged with respect to the receiving body (17).

**4.** Unit (10) according to one of the claims 1-3, wherein the locking surfaces (38) of the locking means are configured in such way that at least four contact points against the sheath (30) are formed.

**5.** Unit (10) according to one of the claims 1-4, wherein each locking surface (38) is attached to an arm (27), said arm (27) being pivotably arranged in such way that the free end of each arm (27) is forced laterally towards the tubular body (17) by means of a skewed contact walls (35) arranged in the housing (18,21), causing a wedging effect so that the locking effects of the knives (38) are further enhanced when the syringed is pulled outwards.

**6.** Unit (10) to one of the claims 1-5, wherein a surface on the receiving body (17) is provided with groove or recess (39) while a corresponding surface of a knob (32) of the housing (18,21) being provided with a pin (33) arranged on a knob (32) movably arranged in said groove (39), the groove (39) being configured in such way the locking effect of the receiver body (17) may be suspended by pushing the receiver body (17) inwards against the action of the spring force and then release the inwards pressure.

**7.** Unit (10) according to one of the claims 1-6, wherein the unit (10) is fixed inside the housing (11,12) configured to be attached to a supporting surface, the unit (10) forming an integral part of the housing (11,12).
